# FASCICULE DE BREVET EUROPEEN

(11) **EP 1 245 580 B1**
(45) Date de publication et mention de la délivrance du brevet: **10.01.2007**
(21) Numéro de dépôt: 02290751.3
(22) Date de dépôt: 26.03.2002
(51) Int. Cl.: C08B 30/18, A23L 1/09, A23L 1/29

(54) **Composition pour nutrition enterale comprenant des fibres**
Faserhaltiges Präparat zur enteralen Ernährung
Fibre containing composition for enteral nutrition

(30) Priorité: 30.03.2001 FR 0104413
(43) Date de publication de la demande: 02.10.2002
(73) Titulaire: Roquette Frères, 62136 Lestrem (FR)
(72) Inventeur: Saniez, Marie-Hélène, 59350 Saint Andre (FR)
(74) Mandataire: Boulinguiez, Didier

(56) Documents cités:
- EP-A- 1 006 128
- WO-A-95/02969
- US-A- 5 952 314
- "Product Specification & Labels" INTERNET, [en ligne] XP002185032 Extrait de l'Internet: <URL:WWW.matsutani.com/fibersol2pdspec.htm l> [extrait le 2002-06-24]

## Description

L'invention se rapporte à une composition pour nutrition entérale comprenant des fibres.

Plus précisément, l'invention a pour objet une composition pour nutrition entérale comprenant des maltodextrines branchées.

La nutrition artificielle permet chez l'adulte comme chez l'enfant, de suppléer un intestin défaillant, de mettre au repos un intestin inflammatoire, de corriger une dénutrition sévère chez un malade ne pouvant ou ne voulant plus s'alimenter, soit du fait d'une affection grave, soit du fait d'un traitement lourd. Sa durée peut s'étaler de quelques jours à quatre semaines à plusieurs mois ou années. Selon les cas, la nutrition artificielle sera effectuée par la voie digestive (nutrition entérale) ou par la voie veineuse (nutrition parentérale). Toujours mise en route en milieu hospitalier, en médecine, chirurgie et réanimation, la nutrition artificielle peut maintenant être continuée à domicile dans les cas nécessitant un traitement très prolongé. La nutrition artificielle, entérale ou parentérale, a pour principe de base l'apport de tous les nutriments nécessaires à la vie : sucres, graisses, protéines, minéraux, oligo-éléments, vitamines, que le sujet ne peut plus prendre par la voie orale habituelle.

La nutrition entérale est la voie la plus simple et la plus physiologique d'assistance nutritive. Le lieu de délivrance est en général intragastrique, à l'aide d'une sonde introduite par voie nasale. Une nutrition entérale efficace et bien tolérée implique deux principes techniques fondamentaux : la lenteur (1 à 3 ml/min) et le caractère continu (de 12h à 24h par jour) de l'instillation alimentaire.

En cas de lésions intestinales étendues compromettant les fonctions d'absorption, les aliments utilisés sont prédigérés (acides aminés, monosaccharides).

Lorsque l'intestin grêle est encore fonctionnel, on utilise une alimentation semi-élémentaire composée de nutriments moins dégradés, voire non dégradés. Les minéraux, oligo-éléments et vitamines sont systématiquement et quotidiennement ajoutés. Les nutriments sont préparés, mélangés et conservés dans des conditions de stricte propreté, voire d'asepsie en cas de préparation industrielle.

Les patients hospitalisés et en particuliers les patients sous antibiotiques ou souffrant de troubles intestinaux, se traduisant soit par une constipation, soit par des diarrhées et qui reçoivent une assistance nutritionnelle par voie entérale ont besoin d'un apport de fibres appropriées.

Les fibres sont reconnues pour leurs effets bénéfiques sur la santé de l'homme et devraient faire partie intégrante des apports alimentaires journaliers. Les fibres sont généralement classées en deux catégories : les fibres solubles et les fibres insolubles. Les fibres solubles, comme la pectine, l'inuline, les amidons résistants sont fermentés par la flore bactérienne intestinale. Cette fermentation libère des acides gras à courte chaîne dans le colon, qui ont pour effet de diminuer le pH de celui-ci et par voie de conséquence de limiter le développement de bactéries pathogènes.

Les fibres insolubles, comme la cellulose, les fibres de maïs ou de soja ont un rôle essentiellement mécanique dans le tractus gastro-intestinal. Elles ne sont que très peu fermentées par la flore intestinale et contribuent à la réduction de la durée du transit intestinal.

Une composition enrichie en fibres pour nutrition entérale doit idéalement :
- être similaire aux fibres classiquement consommées ;
- générer une production d'acides gras volatils bénéfiques ;
- ne pas générer de flatulences chez le patient ;
- améliorer la motricité intestinale ;
- ne pas présenter de résidus ;
- être soluble ;
- présenter une viscosité correcte de manière à faciliter son instillation ;
- être stable au stockage et à la stérilisation ;
- être assimilée progressivement ;
- favoriser l'absorption minérale.

Plusieurs compositions pour nutrition entérale contenant des fibres ont été proposées. Les fibres de pectine ont été proposées mais présentent des inconvénients tels qu'une viscosité trop importante, et une absence d'amélioration de l'absorption minérale.

Le brevet EP-B1 0 756828 décrit des compositions enrichies en fibres, contenant un mélange de fibres solubles, insolubles et d'amidon résistant. Ces compositions contiennent une forte proportion de fibres solubles et présentent une viscosité importante inadaptée à l'instillation par sonde. Ces compositions présentent également des problèmes de stabilité.

Le document EP-A1 1 010 374 décrit un mélange de fibres pour nutrition entérale comprenant des fibres solubles et insolubles de pois, de l'inuline et des fructo-oligo-saccharides. Ces compositions sont également trop visqueuses surtout en raison de la présence de fibres de pois et d'inuline peu solubles, les fructo-oligo-saccharides ne sont pas stables à la stérilisation. Cette instabilité se traduit par une hydrolyse progressive, qui génère une libération de glucose et de fructose et une coloration indésirables. Ces mélanges de fibres ne favorisent que pour partie l'absorption minérale, et ne sont pas assimilées progressivement. D'autre part, les fibres de pois et l'inuline génèrent des problèmes de flatulence chez le patient. Quant aux fructo-oligo-saccharides, ces molécules restent mal tolérées par l'organisme, s'exprimant par la survenue de diarrhées.

Le document US 5 952 314 décrit une composition nutritionnelle destinée aux patients atteints de maladies intestinales, et en particulier de colites ulcératives. Des carbohydrates indigestibles sont incorporés à cette composition de manière à augmenter la production intestinale d'acides gras volatils. Les carbohydrates indigestibles préférés selon ce document sont les fructooligosaccharides, la gomme arabique, la pectine et les polysaccharide issus du soja.

La présente invention a donc pour but de remédier aux inconvénients de l'art antérieur. La Demanderesse a en effet trouvé que l'incorporation desdites maltodextrines branchées à une composition pour nutrition entérale permettait avantageusement de concilier tous ces objectifs jusqu'alors réputés inconciliables en imaginant et élaborant, au prix de nombreuses recherches une composition nouvelle pour nutrition entérale enrichie en fibres qui réponde tous les critères précités, à savoir une viscosité, une solubilité, une stabilité au stockage et à la stérilisation satisfaisante s'exprimant par une absence de rétrogradation, une assimilation progressive, une absence de génération de flatulences, et une amélioration considérable de l'absorption minérale, et en particulier du calcium et du magnésium.

L'invention a donc pour objet une composition pour nutrition entérale enrichie en fibres, caractérisée en ce qu'elle comprend des maltodextrines branchées présentant entre 15 et 35% de liaisons glucosidiques 1-6, une teneur en sucres réducteurs inférieure à 20%, un indice de polymolécularité inférieur à 5 et une masse moléculaire en nombre Mn au plus égale à 4500 g/mole.

Par maltodextrines branchées on entend au sens de la présente invention les maltodextrines décrites dans la demande de brevet EP 1.006.128 dont la Demanderesse est titulaire. Ces maltodextrines branchées présentent un caractère d'indigestibilité qui a pour conséquence de diminuer leur pouvoir calorique, en empêchant leur assimilation au niveau de l'intestin grêle. Elles représentent donc une source de fibres indigestibles, bénéfiques pour le métabolisme et pour l'équilibre intestinal. A titre indicatif, leur taux de fibres insolubles est généralement supérieur à 50% sur matière sèche. Leur teneur élevée en liaisons glucosidiques 1-6 leur confère des propriétés prébiotiques tout à fait particulières : il est en effet apparu que les bactéries du cæcum et du colon de l'homme et des animaux, telles que les bactéries butyrogènes, lactiques ou propioniques métabolisent des composés hautement branchés. D'autre part, ces maltodextrines branchées favorisent le développement des bactéries bifidogènes au détriment des bactéries indésirables. Il en résulte des propriétés tout à fait bénéfiques pour la santé du consommateur. Enfin, le caractère branché desdites maltodextrines diminue considérablement et avantageusement leur tendance à rétrograder, ce qui permet d'envisager leur utilisation dans des applications où l'absence de rétrogradation est nécessaire, en particulier lors d'un stockage prolongé en solution aqueuse.

Toutes les compositions de maltodextrines branchées décrites dans la demande de brevet EP 1.006.128 sont appropriées à la préparation de compositions pour nutrition entérale selon l'invention.

Selon une variante préférée, celles-ci présentent une teneur en sucres réducteurs comprise entre 2 et 5% et une masse moléculaire en nombre comprise entre 2000 et 3000 g/mole.

Selon une autre variante avantageuse, tout ou partie de ces maltodextrines branchées sont hydrogénées, elles sont ainsi avantageusement encore plus stables à la stérilisation.

Une composition pour nutrition entérale enrichie en fibres conforme à l'invention comprend 0,5 à 20 %, de préférence 1 à 10% et plus préférentiellement encore 1 à 5% en poids sec de maltodextrines branchées de manière à constituer un apport de fibres et un apport énergétique suffisant. Les maltodextrines branchées présentent un taux de fibres insolubles supérieur à 50% sur sec, déterminé selon la méthode AOAC N° 985-29 (1986). En deçà de 0,5 % en poids de maltodextrines dans la composition pour nutrition entérale conforme à l'invention, l'apport de fibres est insuffisant pour avoir un effet détectable.

L'amélioration de l'absorption apparente du calcium et du magnésium a été montrée notamment chez l'animal et se traduit par des résultats remarquables comme il sera exemplifié par ailleurs, permettant d'envisager la préparation de compositions pour nutrition entérale améliorant le statut calcique et magnésique des malades. Ceci est surprenant et inattendu pour des composés riches en fibres dont on connaît l'influence négative sur l'absorption des éléments minéraux.

Il a d'autre part été trouvé que les maltodextrines branchées selon l'invention ne généraient pas de diarrhées osmotiques et ce même à des doses importantes. Le phénomène de diarrhées osmotiques est observé lors de l'absorption de carbohydrates fermentescibles de faible poids moléculaire, tels que par exemple le lactulose ou les fructo-oligo-saccharides. Ce phénomène se traduit par une augmentation de la teneur en eau des selles en réaction à une augmentation de l'osmolarité du contenu fécal, cette augmentation de la teneur en eau pouvant aller jusqu'à l'apparition de diarrhées. De manière surprenante et inattendue, les maltodextrines branchées conformes à l'invention ne génèrent pas ce phénomène bien qu'elles soient fermentescibles.

La composition pour nutrition entérale conforme à l'invention comprend également une source de protéines, de lipides et de carbohydrates. De préférence, les protéines représentent environ 10 à 20% de l'apport énergétique de la composition. Elles sont par exemple apportées par des protéines animales comme les protéines de lait, ou des protéines de soja ou encore des protéines d'origine céréalière. Elles peuvent se présenter sous forme native ou hydrolysées de manière à apporter une source d'acides aminés libres.

Les lipides constituent de préférence 30 à 50% de l'apport énergétique de la composition, sous forme d'acides gras monoinsaturés, polyinsaturés et saturés. Toutes les huiles conviennent bien, comme par exemple les huiles d'olive, de maïs, de tournesol, de blé, de raisin.

Les carbohydrates, autres que lesdites maltodextrines branchées représentent de préférence 35 à 55% de l'apport énergétique. Ils peuvent être apportés par des amidons standards ou modifiés, des amidons riches en amylose modifiés ou non, des maltodextrines standard, des sucres simples. Les maltodextrines branchées selon l'invention peuvent bien entendu constituer à la fois un apport énergétique et un apport de fibres.

La composition pour nutrition entérale selon l'invention peut également être complétée d'un apport vitaminique et minéral complet. Des arômes, édulcorants et autre additifs peuvent aussi être ajoutés.

Bien que ce ne soit pas une variante préférée de l'invention, lesdites maltodextrines branchées peuvent être remplacées en tout ou partie par des dextrines indigestibles telles que notamment la FIBERSOL^{®} commercialisée par la société MATSUTANI. Toutefois, ces dernières étant pauvres en fibres insolubles, elles sont avantageusement mélangées avec lesdites maltodextrines branchées.

C'est pourquoi la composition pour nutrition entérale conforme à l'invention peut comprendre des maltodextrines branchées mélangées avec des dextrines indigestibles.

Ladite composition peut se présenter sous une forme prête à l'emploi, c'est à dire sous une forme susceptible d'être instillée au moyen d'une sonde gastrique, ou encore sous forme de boisson comme un jus de fruit, une soupe ou un milk-shake, ou encore sous forme de crèmes desserts ou yaourts directement consommés par le malade ou susceptible d'être administrée par sonde.

Ladite composition peut également se présenter sous forme de poudre à reconstituer dans un liquide.

Les maltodextrines branchées selon l'invention présentent une stabilité en milieu acide et une stabilité aux traitements thermiques tout à fait avantageuses lorsque l'on veut préparer des compositions pour nutrition entérale à pH légèrement acide à acide, comme par exemple des boissons, soupes ou jus de fruits. Cette stabilité n'a pas été observée pour les fructo-oligo-saccharides. De plus, lorsque l'on prépare des compositions destinées à être stérilisées, on préfère souvent que ces compositions soient légèrement acides de façon à éviter la caramélisation des sucres lors du traitement thermique. La stabilité des maltodextrines branchées en milieu acide et/ou à chaud est donc tout à fait intéressante et appropriée.

La composition pour nutrition entérale, qu'elle soit solide ou liquide, sera préalablement stérilisée par toute technique connue de l'homme de l'art et conditionnée si besoin dans des boites, poches ou flacons stériles.

La préparation en elle-même de la composition pour nutrition entérale liquide ou solide peut se faire par toute technique conventionnelle, consistant par exemple en un simple mélange des différents ingrédients en poudre, ou en une atomisation d'une solution comprenant tous lesdits ingrédients. Lorsqu'il s'agit d'une forme liquide, ladite composition doit être facilement administrable au moyen d'une sonde, soit par simple gravité, soit par pompage. Elle devra alors présenter une viscosité réduite, par exemple inférieure à 40 mPa.s à température ambiante.

Ladite composition pour nutrition entérale peut également être utilisée chez l'animal.

L'invention sera mieux comprise à la lecture des exemples qui suivent et de la figure qui s'y rapporte et qui se veulent illustratifs et non limitatifs.

### Exemple 1 : mise en évidence de l'absorption minérale.

Un groupe de rats est alimenté par des mélanges d'aliment standard renfermant 5 à 10% en poids sec de maltodextrines branchées conformes à l'invention. Un autre groupe témoin reçoit l'aliment standard.

Le groupe 1 reçoit l'aliment standard. Le groupe 2 reçoit un aliment mélangé avec 5% de maltodextrines branchées, et le groupe 3 reçoit un aliment mélangé avec 10% de maltodextrines branchées. L'essai est mené pendant 14 jours, au terme desquels un bilan calcium et magnésium est effectué par analyse du calcium et magnésium dans les selles, dans l'aliment et dans l'eau de boisson distribuée aux animaux.

On obtient les résultats suivants :

| | CALCIUM | | MAGNESIUM | |
|---|---|---|---|---|
| | ABSORPTION INTESTINALE* (%) | VARIATION PAR RAPPORT AU GROUPE 1 (%) | ABSORPTION INTESTINALE | VARIATION PAR RAPPORT AU GROUPE 1 (%) |
| GROUPE 1 | 21,52 | - | 32,74 | - |
| GROUPE 2 | 29,62 | + 37,6 | 39,34 | + 20,2 |
| GROUPE 3 | 38,49 | + 78,9 | 47,8 | + 46,0 |

| | | | | |
|---|---|---|---|---|
| * l'absorption intestinale est calculée par soustraction du taux du minéral considéré dans les selles au taux du même minéral dans l'aliment et dans la boisson, exprimée en pourcentage. | | | | |

Ces résultats démontrent clairement l'influence tout à fait positive d'un apport de maltodextrines branchées à l'alimentation des rats sur l'absorption des minéraux dans le tractus digestif, ce qui est tout à fait surprenant pour des composés riches en fibres dont on connaît leurs effets défavorables sur l'absorption des éléments minéraux.

### Exemple 2 : préparation d'une composition pour nutrition entérale selon l'invention.

Une composition pour nutrition entérale est préparée en mélangeant dans de l'eau distillée des maltodextrines branchées de composition suivante :

| | |
|---|---|
| Sucres réducteurs | 2,3 |
| Mn (g/mole) | 2480 |
| Mw (g/mole) | 5160 |
| Liaison 1,2 (%) | 10 |
| Liaison 1,3 (%) | 12 |
| Liaison 1,4 (%) | 49 |
| Liaison 1,6 (%) | 29 |

A cette solution sont ajoutés un mélange d'huiles de mais, de colza et de soja, un mélange de protéines de lait et de soja, une maltodextrine standard, ainsi que des vitamines et minéraux.

La composition obtenue est décrite ci-après (% en poids) :
- protéines : 4%
- carbohydrates : 11%
- lipides : 4%
- maltodextrines branchées : 3%
- fibres insolubles : 1,5%

La composition obtenue est stockée pendant six mois, après quoi la stabilité au stockage est évaluée : la composition est homogène et ne présente pas de cristaux ou sédiments, traduisant une absence de rétrogradation et donc une bonne stabilité au stockage, et permettant d'envisager pour les compositions conformes à l'invention une date de péremption supérieure à six mois.

### Exemple 3 : étude de stabilité en solution.

Afin d'estimer la stabilité des maltodextrines branchées dans des boissons, on prépare des solutions à différents pH, contenant soit des fructo-oligo-saccharides (ACTILIGHT 950P commercialisé par BEGHIN-SAY ou RAFTILOSE P35 commercialisé par ORAFTI), soit des maltodextrines branchées conformes à l'invention.

On mesure au cours du stockage l'évolution des masses moléculaires de ces oligosaccharides en solution, par exclusion stérique suivie d'une détection par réfractométrie différentielle.

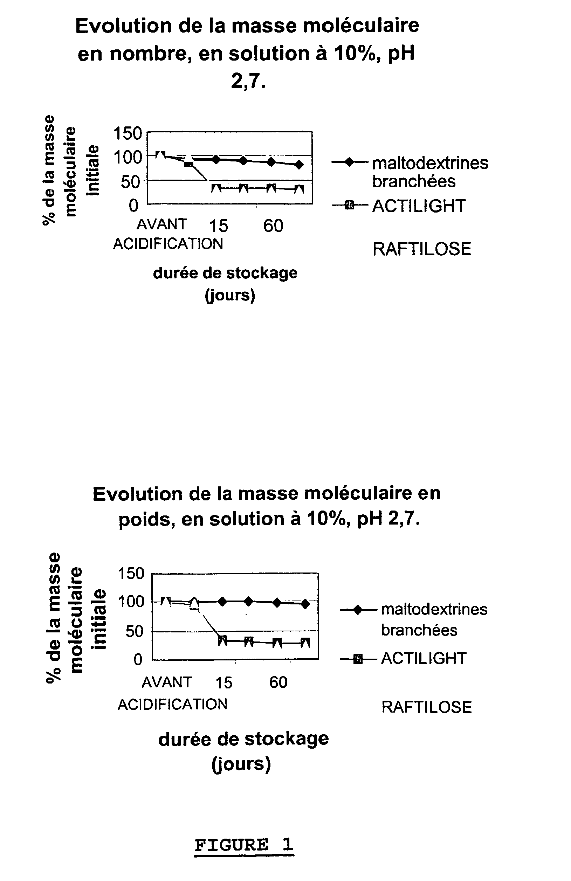

Les résultats sont illustrés en figure 1.

Dans le cas des maltodextrines branchées, on constate une très légère répercussion de l'acidification des solutions. Il y a donc une légère hydrolyse aux pH très acides (inférieurs à 3) qui se traduit par une évolution du poids moléculaire vers des valeurs plus basses. Après deux semaines de stockage, les différences ne sont pas supérieures à celles observées immédiatement après acidification. Après 1 mois de stockage, seules les valeurs obtenues pour les pH très faibles (inférieurs à 2,7) ont diminué par rapport au vieillissement de deux semaines. On passe d'un poids moléculaire en nombre de 2800 daltons à 2400 daltons pour des solutions à pH 2.

Dans le cas des fructo-oligo-saccharides, après deux semaines de stockage à pH 2 le poids moléculaire est réduit à 30% de sa valeur initiale. Après 1 mois, les solutions à pH 2 et 2,7 contiennent des fructo-oligo-saccharides très fortement dégradés.
Conclusion : les maltodextrines branchées sont bien adaptées à la préparation de boissons, de jus de fruits ou de légumes, présentant un pH généralement inférieur à 3.

### Exemple 4 : stabilité aux traitements thermiques

On prépare des jus de fruits comprenant des maltodextrines branchées selon l'invention, que l'on soumet à un traitement de pasteurisation pendant 17 minutes à 74°C. On prépare également un témoin sans maltodextrines branchées et un témoin comprenant des fructo-oligo-saccharides. On compare les teneurs avant et après traitement, ainsi qu'après stockage. Tous les jus de fruits on un pH de 3,8.

Les résultats sont donnés par le tableau suivant :

| | TEMOIN | 7% maltodextrines branchées | | 7% maltodextrines branchées hydrogénées | | 7% fructo-oligo-saccharides (ACTILIGHT^{®}) | |
|---|---|---|---|---|---|---|---|
| | Saccharose | Saccharose | **Malt. branch.** | Saccharose | **Malt. branch. hydrogé nées** | Saccharose | FOS |
| Avant pasteuri sation | 2,5 | 2,4 | 7,0 | 2,1 | 6,8 | 2,3 | 6,7 |
| Après pasteuri sation | 1,4 | 1,6 | 7,0 | 1,6 | 6,8 | 2,4 | 4,5 |
| Après pasteuri sation + 1,5 mois | nd | nd | 6,9 | nd | 6,9 | 3,8 | 2,4 |
| Après pasteuri sation + 3 mois | 1,5 | 1,5 | 7,1 | 1,2 | 7,1 | 3,4 | 1,7 |

Ces résultats démontrent l'absence de dégradation des maltodextrines branchées selon l'invention, hydrogénées ou non, car elles ne sont hydrolysées ni au cours du traitement thermique, ni au cours du stockage pendant trois mois. Les fructo-oligosaccharides se dégradent fortement avec la pasteurisation (-30%) et avec la conservation (-60% après trois mois par rapport à la valeur après pasteurisation). Ceci est observé à la fois par la diminution du taux de FOS et par l'augmentation de la teneur en saccharose (produit d'hydrolyse). Ceci confirme l'intérêt tout particulier des maltodextrines selon l'invention dans des compositions pour nutrition entérale destinées en particulier à être stérilisées ou pasteurisées.

## Revendications

1. Composition pour nutrition entérale enrichie en fibres **caractérisée en ce qu'**elle comprend 0,5 à 20%, de préférence 1 à 10% et plus préférentiellement encore 1 à 5% en poids sec de maltodextrines branchées présentant entre 15 et 35% de liaisons glucosidiques 1→6, une teneur en sucres réducteurs inférieure à 20%, un indice de polymolécularité inférieur à 5 et une masse moléculaire moyenne en nombre Mn au plus égale à 4500 g/mole.

2. Composition pour nutrition entérale enrichie en fibres selon la revendication 1, **caractérisée en ce que** lesdites maltodextrines branchées présentent une teneur en sucres réducteurs comprise entre 2 et 5% et une masse moléculaire moyenne en nombre Mn comprise entre 2000 et 3000 g/mole.

3. Composition pour nutrition entérale enrichie en fibres selon l'une ou l'autre des revendications 1 et 2, **caractérisée en ce que** tout ou partie desdites maltodextrines branchées est hydrogénée.

4. Composition pour nutrition entérale selon l'une quelconque des revendications 1 à 3, **caractérisée en ce qu'**elle comprend en outre des protéines, et/ou des lipides, et/ou des carbohydrates, des vitamines et des minéraux.

5. Composition pour nutrition entérale selon l'une quelconque des revendications 1 à 4 **caractérisée en ce qu'**elle est une poudre à reconstituer.

6. Composition pour nutrition entérale selon l'une quelconque des revendications 1 à 4 **caractérisée en ce qu'**elle est une composition prête à l'emploi.

7. Composition pour nutrition entérale selon l'une quelconque des revendications 1 à 6 **caractérisée en ce que** lesdites maltodextrines branchées présentent un taux de fibres insolubles supérieur à 50% sur matière sèche.

8. Composition pour nutrition entérale selon l'une quelconque des revendications 1 à 7 **caractérisée en ce que** lesdites maltodextrines branchées sont mélangées avec des dextrines indigestibles.

9. Composition pour nutrition entérale selon l'une quelconque des revendications 1 à 8, **caractérisée en ce que** lesdites maltodextrines branchées sont stables en conditions acides et/ou à chaud.

## Claims

1. Fibre-enriched composition for enteral nutrition, **characterized in that** it comprises 0.5 to 20%, preferably 1 to 10%, and still more preferably 1 to 5% by dry weight of branched maltodextrins having between 15 and 35% of 1→6 glucoside linkages, a reducing sugar content of less than 20%, a polymolecularity index of less than 5 and a number-average molecular mass Mn at most equal to 4500 g/mol.

2. The fibre-enriched composition for enteral nutrition of claim 1, wherein said branched maltodextrins have a reducing sugar content of between 2 and 5% and a number-average molecular mass Mn of between 2000 and 3000 g/mol.

3. The fibre-enriched composition for enteral nutrition of any one of claims 1 and 2, wherein all or some of said branched maltodextrins are hydrogenated.

4. The fibre-enriched composition for enteral nutrition of any one of claims 1 to 3, which further comprises proteins, and/or lipids, and/or carbohydrates, vitamins and minerals.

5. The fibre-enriched composition for enteral nutrition of any one of claims 1 to 4, which is a powder to be reconstituted.

6. The fibre-enriched composition for enteral nutrition of any one of claims 1 to 4, which is a ready-to-use composition.

7. The fibre-enriched composition for enteral nutrition of any one of claims 1 to 6, wherein said branched maltodextrins have an insoluble fibre level greater than 50% on a dry matter basis.

8. The fibre-enriched composition for enteral nutrition of any one of claims 1 to 7, wherein said branched maltodextrins are mixed with indigestible dextrins.

9. The fibre-enriched composition for enteral nutrition of any one of claims 1 to 8, wherein said branched maltodextrins are stable under acidic conditions and/or to heat.

## Patentansprüche

1. Mit Fasern angereicherte Zusammensetzung für die enterale Ernährung, **dadurch gekennzeichnet, dass** sie 0,5 bis 20 Gewichts-%, vorzugsweise 1 bis 10 Gewichts-% und besonders bevorzugt 1 bis 5 Gewichts-% verzweigte Maltodextrine, die zwischen 15 und 35% 1,6-glykosidische Verbindungen aufweisen, einen Gehalt an reduzierenden Zuckern von unter 20%, einen Polymolekularitätsindex von unter 5% und eine anzahlbezogene mittlere Molekularmasse Mn von höchstens 4500 g/Mol aufweist.

2. Mit Fasern angereicherte Zusammensetzung für die enterale Ernährung nach Anspruch 1, **dadurch gekennzeichnet, dass** die verzweigten Maltodextrine einen Gehalt an reduzierenden Zuckern von zwischen 2 und 5% und eine anzahlbezogene mittlere molekulare Masse zwischen 2000 und 3000 g/Mol aufweisen.

3. Mit Fasern angereicherte Zusammensetzung für die enterale Ernährung nach Anspruch 1 oder 2, **dadurch gekennzeichnet, dass** die gesamten Maltodextrine oder ein Teil von ihnen hydriert ist.

4. Zusammensetzung für die enterale Ernährung nach einem beliebigen der Ansprüche 1 bis 3, **dadurch gekennzeichnet, dass** sie ferner Proteine und/oder Lipide und/oder Kohlenhydrate, Vitamine und Mineralien enthält.

5. Zusammensetzung für die enterale Ernährung nach einem beliebigen der Ansprüche 1 bis 4, **dadurch gekennzeichnet, dass** sie ein zu rekonstituierendes Pulver ist.

6. Zusammensetzung für die enterale Ernährung nach einem beliebigen der Ansprüche 1 bis 4, **dadurch gekennzeichnet, dass** sie eine gebrauchsfertige Zusammensetzung ist.

7. Zusammensetzung für die enterale Ernährung nach einem beliebigen der Ansprüche 1 bis 6, **dadurch gekennzeichnet, dass** die verzweigten Maltodextrine einen Trockenmasseanteil von über 50% an unlöslichen Fasern aufweisen.

8. Zusammensetzung für die enterale Ernährung nach einem der beliebigen Ansprüche 1 bis 7, **dadurch gekennzeichnet, dass** die verzweigten Maltodextrine mit unverdaulichen Dextrinen gemischt sind.

9. Zusammensetzung für die enterale Ernährung nach einem beliebigen der Ansprüche 1 bis 8, das die verzweigten Maltodextrine unter sauren Bedingungen und/oder unter Hitze stabil sind.
